(19) **Europäisches Patentamt European Patent Office Office européen des brevets**

(11) **EP 4 775 979 A1**

(12) **EUROPEAN PATENT APPLICATION**
published in accordance with Art. 153(4) EPC

(43) Date of publication:
15.07.2026 Bulletin 2026/29

(21) Application number: 25801850.6

(22) Date of filing: 25.06.2025

(51) International Patent Classification (IPC):
$G01N\ 30/02^{(2006.01)}$ $G01N\ 30/72^{(2006.01)}$
$G01N\ 30/86^{(2006.01)}$ $G06F\ 17/11^{(2006.01)}$

(86) International application number:
PCT/CN2025/103355

(87) International publication number:
WO 2026/077008 (16.04.2026 Gazette 2026/16)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 08.10.2024 CN 202411391050

(71) Applicant: China Tobacco Yunnan Industrial Co.,
Ltd.
Kunming, Yunnan 650024 (CN)

(72) Inventors:
• YANG, Lei
Kunming, Yunnan 650032 (CN)
• ZHANG, Tao
Kunming, Yunnan 650032 (CN)
• LI, Chao
Kunming, Yunnan 650032 (CN)
• YE, Xiangrui
Kunming, Yunnan 650032 (CN)

• ZHANG, Tao
Kunming, Yunnan 650032 (CN)
• YE, Yin
Kunming, Yunnan 650032 (CN)
• HE, Xuefeng
Kunming, Yunnan 650032 (CN)
• GUAN, Bin
Kunming, Yunnan 650032 (CN)
• YANG, Yanyang
Kunming, Yunnan 650032 (CN)
• ZHANG, Ling
Kunming, Yunnan 650032 (CN)
• JIANG, Mengfei
Kunming, Yunnan 650032 (CN)
• YANG, Xin
Kunming, Yunnan 650032 (CN)

(74) Representative: Fezzardi, Antonio et al
Studio Ferrario Srl
Via Collina, 36
00187 Roma (IT)

(54) **METHOD FOR EVALUATING SPICY AROMA NOTE OF CIGARETTE SMOKE, AND APPLICATION OF METHOD**

(57) Provided are an evaluation method for the spicy aroma note of cigarette smoke and use thereof. The evaluation method comprises the following steps: (1) collecting a mainstream smoke total particulate matter of a typical spicy-type cigarette and a typical non-spicy-type cigarette, performing GC-MS detection to obtain a content of an aroma-contributing component; and conducting partial least squares-discriminant analysis on the content of the aroma-contributing component to obtain a VIP value of the aroma-contributing component; (2) collecting a mainstream smoke total particulate matter of a cigarette to be tested, and performing GC-MS detection to obtain a content of an aroma-contributing component in the cigarette to be tested; and (3) calculating a spicy aroma note index Q based on the VIP value and the content of the aroma-contributing component in the cigarette to be tested. The evaluation method provided in the present application can objectively and accurately reflect the spicy aroma note of cigarette smoke, avoiding the subjective errors inherent in sensory evaluation and the mismatch between the analysis result of aroma-contributing components in cut tobacco and the actual smoking experience, thereby providing a novel approach for characterizing and evaluating the spicy aroma note of cigarette smoke.

EP 4 775 979 A1

**Description**

TECHNICAL FIELD

The present application belongs to the technical field of tobacco analysis, and specifically relates to an evaluation method for the spicy aroma note of cigarette smoke and use thereof.

BACKGROUND

The consumption pattern of cigarette products by consumers determines that the characteristic aroma notes of the cigarette smoke are decisive in defining a cigarette's style, serving as one of the most critical foundations for product category development. Relevant studies have shown that the overall aroma profile of a cigarette is primarily formed by the combined interaction of its various characteristic aroma notes. Furthermore, each characteristic aroma note is underpinned by a distinct set of materials that make significant contributions. At present, sensory evaluation remains the cornerstone of the characteristic aroma note assessment system. However, this method lacks support from objective data and is relatively time-consuming and labor-intensive to implement. At the molecular level of the material basis, researchers in the industry have characterized aroma profiles based on aroma-contributing components in cut tobacco using indices, which has a certain positive effect in evaluating aroma characteristics. However, this approach does not fully account for the transfer rate of aroma-contributing components from the cut tobacco into the cigarette smoke, or the changes in types and contents of aroma-contributing components during the combustion process of cigarettes. This is particularly critical for the spicy aroma note, as it can significantly influence consumers' sensory evaluation of cigarette products, making its assessment especially important.

Therefore, it is particularly important to utilize instrumental analysis and detection methods, combined with multivariate statistical analysis techniques, and based on aroma-contributing components of cigarette smoke, to identify and extract a set of characteristic aroma-contributing components that significantly contribute to the spicy aroma note of cigarette smoke from various chemical components, so as to objectively and accurately characterize and evaluate the characteristic aroma notes of cigarette smoke.

SUMMARY

The following is a brief summary of subject matter that is described in greater detail herein. This summary is not intended to be limiting as to the scope of the claims.

In view of the shortcomings of existing technologies, an object of the present application is to provide an evaluation method for the spicy aroma note of cigarette smoke and use thereof. The evaluation method provided in the present application can objectively and accurately reflect the spicy aroma note of cigarette smoke, avoiding the subjective errors inherent in sensory evaluation and the mismatch between the analysis result of aroma-contributing components in cut tobacco and the actual smoking experience. This method provides a novel approach for characterizing and evaluating the spicy aroma note of cigarette smoke, playing a significantly positive role in the rapid assessment of the spicy aroma note of cigarette smoke and research on the enhancement of the spicy character of cigarettes.

To achieve the object, the present application adopts the following technical solution.

On one hand, the present application provides an evaluation method for the spicy aroma note of cigarette smoke, and the evaluation method comprises the following steps:

(1) collecting a mainstream smoke total particulate matter of a typical spicy-type cigarette and a typical non-spicy-type cigarette, performing GC-MS (gas chromatography-mass spectrometry) detection to obtain a content of an aroma-contributing component in the typical spicy-type cigarette and the typical non-spicy-type cigarette; and conducting partial least squares-discriminant analysis on the content of the aroma-contributing component to obtain a VIP value (variable importance in projection value) of the aroma-contributing component;

(2) collecting a mainstream smoke total particulate matter of a cigarette to be tested, and performing GC-MS detection to obtain a content of an aroma-contributing component in the cigarette to be tested; and

(3) calculating a spicy aroma note index Q for cigarette smoke of the cigarette to be tested based on the VIP value of the aroma-contributing component from step (1) and the content of the aroma-contributing component in the cigarette to be tested obtained in step (2), and determining a degree of the spicy aroma note of cigarette smoke of the cigarette to be tested;

steps (1) and (2) are not required to be performed in a particular order.

The aroma-contributing component comprises a positively correlated aroma-contributing component and a negatively correlated aroma-contributing component.

The positively correlated aroma-contributing component comprises any one or a combination of at least two of a terpenoid aroma-contributing component, a long-chain unsaturated fatty acid methyl ester, and a positively correlated ketonic aroma-contributing component. The negatively correlated aroma-contributing component comprises any one or a combination of at least two of a nitrogen-containing heterocyclic aroma-contributing component, a furanic aroma-contributing component, and a negatively correlated ketonic aroma-contributing component.

In the above method, by analyzing the aroma-contributing component in the typical spicy-type cigarette, the typical non-spicy-type cigarette, and the cigarette to be tested, utilizing the partial least squares-discriminant analysis to obtain the VIP value, and combining with the content of the aroma-contributing component to calculate the spicy aroma note index Q, the spicy aroma note of cigarette smoke can be objectively and accurately reflected, avoiding the subjective errors inherent in sensory evaluation and the mismatch between the analysis result of aroma-contributing components in cut tobacco and the actual smoking experience, providing a novel approach for characterizing and evaluating the spicy aroma note of cigarette smoke, and playing a significantly positive role in the rapid assessment of the spicy aroma note of cigarette smoke and research on the enhancement of the spicy character of cigarettes.

In one embodiment, the positively correlated aroma-contributing component comprises a combination of a terpenoid aroma-contributing component, a long-chain unsaturated fatty acid methyl ester, and a positively correlated ketonic aroma-contributing component.

In one embodiment, the negatively correlated aroma-contributing component comprises a combination of a nitrogen-containing heterocyclic aroma-contributing component, a furanic aroma-contributing component, and a negatively correlated ketonic aroma-contributing component.

In one embodiment, the terpenoid aroma-contributing component comprises neophytadiene.

In one embodiment, the long-chain unsaturated fatty acid methyl ester aroma-contributing component comprises methyl linolenate and/or methyl palmitate.

In one embodiment, the positively correlated ketonic aroma-contributing component comprises 3-hydroxybutan-2-one.

In one embodiment, the nitrogen-containing heterocyclic aroma-contributing component comprises pyridine.

In one embodiment, the furanic aroma-contributing component comprises 2-ethylfuran.

In one embodiment, the negatively correlated ketonic aroma-contributing component comprises cyclopentanone and/or 3-pentene-2-one.

The combination of the above specified aroma-contributing components enables a comprehensive and effective evaluation of the spicy aroma note in cigarette smoke of the cigarette to be tested, significantly enhancing the accuracy of the assessment.

In one embodiment, in the GC-MS detection, a chromatographic column is HP-5MS.

In one embodiment, in the GC-MS detection, a carrier gas flow rate is 0.8-1.2 mL/min, for example, 0.8 mL/min, 0.9 mL/min, 1 mL/min, 1.1 mL/min, or 1.2 mL/min, but is not limited to the listed values, and other unlisted values within the numerical range are also applicable.

In one embodiment, in the GC-MS detection, a temperature program is as follows:
setting 40-60°C as an initial temperature and maintaining for 0.7-1.3 min; heating to 155-175°C at a rate of 6-10°C/min and maintaining for 1.4-2.4 min; and then heating to 255-275°C at a rate of 6-10°C/min and maintaining for 12-16 min.

The above specific GC-MS detection conditions can effectively improve the accuracy of the detection results, and thereby enhance the accuracy of the assessment results.

In one embodiment, a calculation formula of the spicy aroma note index Q in step (3) is as follows:

$$Q = \Sigma(\text{content of positively correlated aroma-contributing component } i \times VIP_i) / \Sigma(\text{content of negatively correlated aroma-contributing component } j \times VIP_j);$$

wherein, $VIP_i$ represents the VIP value of the positively correlated aroma-contributing component i, and $VIP_j$ represents the VIP value of the negatively correlated aroma-contributing component j.

In the above formula, the spicy aroma note of cigarette smoke has a positive correlation to the index Q, that is, a higher Q (spicy aroma note index of smoke of the cigarette to be tested) value indicates a more intense spicy aroma character of the cigarette smoke.

On the other hand, the present application further provides use of the above evaluation method in the evaluation and analysis of aroma note of cigarette smoke.

Compared with the prior art, the present application has the following beneficial effects.

The present application provides an evaluation method for the spicy aroma note of cigarette smoke. By analyzing the aroma-contributing component in the typical spicy-type cigarette, the typical non-spicy-type cigarette, and the cigarette to be tested, utilizing the partial least squares-discriminant analysis to obtain the VIP value, and combining with the content of the aroma-contributing component to calculate the spicy aroma note index Q, the spicy aroma note of cigarette smoke can be objectively and accurately reflected, avoiding the subjective errors inherent in sensory evaluation and the mismatch between the analysis result of aroma-contributing components in cut tobacco and the actual smoking experience, providing a novel approach for characterizing and evaluating the spicy aroma note of cigarette smoke, and playing a significantly positive role in the rapid assessment of the spicy aroma note of cigarette smoke and research on the enhancement of the spicy character of cigarettes.

Other aspects will be appreciated upon reading and understanding the detailed description.

DETAILED DESCRIPTION

To further illustrate the technical schemes employed in the present application and effects thereof, the technical solutions of the present application will be further explained below in conjunction with preferred embodiments of the present application; however, the present application is not limited to the scope of the embodiments.

**Example 1**

This example provides an evaluation method for the spicy aroma note of cigarette smoke, and the specific steps are as follows.

Detection of aroma-contributing component

The mainstream smoke total particulate matter of cigarettes was collected according to the method specified in the Chinese National Standard GB/T 19609-2004 "Cigarette - Determination of total and nicotine-free dry particulate matter using a routine analytical smoking machine".

The collected filter disc was put into a simultaneous distillation-extraction apparatus, and subjected to simultaneous distillation-extraction for 2 h by using dichloromethane as a solvent. The obtained extract was dried with anhydrous sodium sulfate, then placed in a rotary evaporator and concentrated to 1.0 mL, and added with 50 $\mu$L (0.1 mol/L) of benzyl acetate solution in anhydrous ethanol, and shaken well for later GC-MS analysis.

Conditions for GC-MS analysis

Chromatographic column: HP-5MS (30 m × 0.25 mm × 0.25 $\mu$m) capillary column; inlet temperature: 240°C; transmission line temperature: 280°C; carrier gas: He, with a flow rate of 1.0 mL/min; temperature programming: the temperature was maintained at 50°C for 1 min, heated to 165°C at a rate of 8°C/min and maintained for 1.9 min, and then heated to 265°C at a rate of 8°C/min and maintained for 14 min; injection volume: 2 $\mu$L, with a split ratio of 25:1; EI ionization energy: 70 eV; ion source temperature: 230°C; quadrupole temperature: 160°C; mass range: 35-455 amu. The retrieval and qualitative analysis were performed by using the NIST 17 and Wiley 275 standard mass spectral libraries, and the content of partial aroma-contributing components was determined using the internal standard method.

(1) Aroma-contributing components in typical spicy-type cigarettes and typical non-spicy-type cigarettes were detected using the above method. Partial least squares-discriminant analysis (PLS-DA) was performed using a chemometric statistical software SIMCA 14.1. The VIP values of the terpenoid aroma-contributing component (neophytadiene), the long-chain unsaturated fatty acid methyl ester aroma-contributing component (methyl linolenate and methyl palmitate), the ketonic aroma-contributing component (3-hydroxybutan-2-one, cyclopentanone, and 3-pentene-2-one), the nitrogen-containing heterocyclic aroma-contributing component (pyridine), and the furanic aroma-contributing component (2-ethylfuran) were calculated, as shown in the table below.

| Aroma-contributing component | VIP value |
| --- | --- |
| Neophytadiene | 1.658 |
| Methyl linolenate | 1.429 |
| Methyl palmitate | 1.479 |
| 3-Hydroxybutan-2-one | 1.447 |
| Pyridine | 1.248 |
| 2-Ethylfuran | 1.39 |
| Cyclopentanone | 1.668 |

(continued)

| Aroma-contributing component | VIP value |
|---|---|
| 3-Pentene-2-one | 1.545 |

(2) The above method was employed to determine the aroma-contributing components in 38 types of commercially available cigarettes, and the results are shown in the table below.

| Sample Name | Compound name (unit: μg/individual) | | | | | | | |
|---|---|---|---|---|---|---|---|---|
| | Neophytadiene | Methyl linolenate | Methyl palmitate | 3-Hydroxybutan-2-one | Pyridine | 2-Ethyl furan | Cyclopentanone | 3-Pentene-2-one |
| JP-1 | 7.2 | 3.244 | 2.016 | 0.128 | 0.963 | 0.586 | 0.251 | 0.209 |
| JP-2 | 4.158 | 5.161 | 3.278 | 0.142 | 0.748 | 0.561 | 0.187 | 0.187 |
| JP-3 | 3.506 | 2.781 | 1.946 | 0.151 | 1.529 | 1.305 | 0.522 | 0.373 |
| JP-4 | 4.802 | 3.517 | 2.3 | 0.118 | 1.687 | 1.321 | 0.606 | 0.433 |
| JP-5 | 2.088 | 2.075 | 1.342 | 0.105 | 0.787 | 0.445 | 0.205 | 0.171 |
| JP-6 | 5.189 | 4.987 | 3.243 | 0.114 | 1.06 | 0.893 | 0.335 | 0.223 |
| JP-7 | 4.926 | 4.171 | 2.698 | 0.23 | 1.277 | 0.867 | 0.456 | 0.274 |
| JP-8 | 4.686 | 3.39 | 2.098 | 0.118 | 0.891 | 0.581 | 0.232 | 0.21 |
| JP-9 | 4.839 | 4.129 | 2.989 | 0.115 | 1.125 | 0.796 | 0.338 | 0.225 |
| JP-10 | 5.051 | 4.714 | 2.507 | 0.082 | 0.408 | 0.204 | 0.102 | 0.067 |
| JP-11 | 3.513 | 2.621 | 2.105 | 0.093 | 0.867 | 0.547 | 0.205 | 0.187 |
| JP-12 | 3.623 | 2.928 | 2.243 | 0.136 | 1.073 | 0.581 | 0.268 | 0.224 |
| JP-13 | 4.636 | 3.417 | 2.702 | 0.112 | 0.485 | 0.216 | 0.054 | 0.108 |
| JP-14 | 5.756 | 3.674 | 2.073 | 0.15 | 1.255 | 0.701 | 0.406 | 0.258 |
| JP-15 | 6.128 | 5.321 | 3.43 | 0.113 | 0.386 | 0.221 | 0.055 | 0.104 |
| JP-16 | 9.436 | 3.093 | 2.79 | 0.048 | 0.042 | 0.068 | 0.205 | 0.190 |
| JP-17 | 7.459 | 4.531 | 2.956 | 0.117 | 0.463 | 0.231 | 0.116 | 0.08 |
| JP-18 | 4.307 | 3.477 | 2.761 | 0.117 | 0.701 | 0.351 | 0.15 | 0.133 |
| JP-19 | 4.67 | 2.568 | 2.177 | 0.09 | 0.519 | 0.236 | 0.094 | 0.095 |
| JP-20 | 6.969 | 3.316 | 1.787 | 0.116 | 0.53 | 0.303 | 0.114 | 0.061 |
| JP-21 | 4.748 | 5.792 | 3.527 | 0.043 | 0.409 | 0.327 | 0.123 | 0.082 |
| JP-22 | 4.451 | 4.616 | 3.022 | 0.089 | 0.479 | 0.239 | 0.096 | 0.078 |
| JP-23 | 4.624 | 3.167 | 2.343 | 0.145 | 0.906 | 0.667 | 0.286 | 0.191 |
| JP-24 | 5.298 | 3.779 | 2.036 | 0.077 | 0.601 | 0.451 | 0.188 | 0.113 |
| JP-25 | 5.587 | 5.326 | 3.012 | 0.096 | 0.939 | 0.516 | 0.282 | 0.154 |
| JP-26 | 6.112 | 4.424 | 2.572 | 0.087 | 0.812 | 0.427 | 0.256 | 0.171 |
| JP-27 | 2.405 | 3.114 | 1.828 | 0.105 | 0.653 | 0.447 | 0.172 | 0.137 |
| JP-28 | 2.435 | 2.122 | 1.286 | 0.093 | 0.639 | 0.487 | 0.213 | 0.122 |
| JP-29 | 4.134 | 2.485 | 1.803 | 0.086 | 0.919 | 0.526 | 0.292 | 0.167 |
| JP-30 | 8.118 | 5.782 | 3.568 | 0.054 | 0.524 | 0.314 | 0.157 | 0.105 |
| JP-31 | 2.639 | 1.948 | 0.714 | 0.044 | 0.545 | 0.293 | 0.168 | 0.126 |
| JP-32 | 2.17 | 2.38 | 0.95 | 0.096 | 0.802 | 0.66 | 0.273 | 0.189 |

(continued)

| Sample Name | Compound name (unit: μg/individual) | | | | | | | |
|---|---|---|---|---|---|---|---|---|
| | Neophyt adiene | Methyl linolenate | Methyl palmitate | 3-Hydroxyb utan-2-one | Pyridi ne | 2-Ethyl furan | Cyclopen tanone | 3-Penten e-2-one |
| JP-33 | 4.058 | 2.997 | 1.94 | 0.079 | 0.773 | 0.657 | 0.271 | 0.155 |
| JP-34 | 2.165 | 1.399 | 1.024 | 0.1 | 0.853 | 0.558 | 0.215 | 0.157 |
| JP-35 | 3.983 | 2.284 | 2.044 | 0.093 | 1.132 | 0.86 | 0.362 | 0.226 |
| JP-36 | 3.629 | 3.139 | 2.205 | 0.155 | 1.329 | 1.125 | 0.409 | 0.307 |
| JP-37 | 2.305 | 1.811 | 1.008 | 0.093 | 0.94 | 0.618 | 0.334 | 0.243 |
| JP-38 | 5.806 | 4.572 | 2.831 | 0.054 | 0.575 | 0.314 | 0.123 | 0.105 |

(3) The spicy aroma note index Q of cigarette smoke of the above corresponding 38 types of commercially available cigarettes was calculated, and the Q index reflected the spicy aroma note of cigarette smoke; the spicy aroma note index Q of smoke of the cigarette to be tested was calculated using a weighted statistical method, with the formula as follows:

$$Q = \Sigma(\text{content of positively correlated aroma-contributing component } i \times VIP_i) / \Sigma(\text{content of negatively correlated aroma-contributing component } j \times VIP_j).$$

In the above formula, $VIP_i$ referred to the VIP value of the positively correlated aroma-contributing component i, and $VIP_j$ referred to the VIP value of the negatively correlated aroma-contributing component j. The positively correlated aroma-contributing component was neophytadiene, methyl linolenate, methyl palmitate, and 3-hydroxybutan-2-one. The negatively correlated aroma-contributing component was pyridine, 2-ethylfuran, cyclopentanone, and 3-pentene-2-one. The results are shown in the table below.

| Sample name | Q value | Sample name | Q value | Sample name | Q value |
|---|---|---|---|---|---|
| JP-1 | 7.16 | JP-14 | 5.00 | JP-27 | 5.84 |
| JP-2 | 8.35 | JP-15 | 22.09 | JP-28 | 4.51 |
| JP-3 | 2.49 | JP-16 | 31.01 | JP-29 | 5.03 |
| JP-4 | 2.95 | JP-17 | 19.23 | JP-30 | 17.88 |
| JP-5 | 3.88 | JP-18 | 9.00 | JP-31 | 5.30 |
| JP-6 | 5.97 | JP-19 | 11.54 | JP-32 | 3.20 |
| JP-7 | 4.63 | JP-20 | 13.98 | JP-33 | 5.45 |
| JP-8 | 6.04 | JP-21 | 16.52 | JP-34 | 2.97 |
| JP-9 | 5.41 | JP-22 | 15.34 | JP-35 | 3.66 |
| JP-10 | 17.76 | JP-23 | 5.61 | JP-36 | 3.19 |
| JP-11 | 5.18 | JP-24 | 9.28 | JP-37 | 2.71 |
| JP-12 | 4.66 | JP-25 | 8.27 | JP-38 | 13.42 |
| JP-13 | 14.39 | JP-26 | 8.87 | - | - |

(4) To validate the close correlation between the Q value and the intensity trend of the spicy aroma note of cigarette smoke, a grouped comparative sensory evaluation was conducted. 18 groups of samples were randomly selected and distributed according to high, medium, and low Q values. Each group consisted of No. 1, No. 2, and No. 3, with the Q value descending in the order of No. 1 > No. 2 > No. 3. A sensory panel of twelve experts performed the comparative smoking evaluation in accordance with the stipulations of GB 5606.4-2005 "Cigarettes - Technical Requirements for Sense Taste". The results demonstrated that the evaluation conclusions for six groups were consistent with the Q value trends (see table below), indicating the Q value has applicable value in characterizing and evaluating the spicy aroma note of cigarette smoke, and can accurately and effectively evaluate the spicy aroma note of cigarette smoke.

| No. 1 | | No. 2 | | No. 3 | | Sensory evaluation result of spicy aroma note |
|---|---|---|---|---|---|---|
| Sample name | Q value | Sample name | Q value | Sample name | Q value | |
| JP-16 | 31.01 | JP-30 | 17.88 | JP-23 | 5.61 | No.1> No. 2 > No. 3 |
| JP-17 | 19.23 | JP-14 | 5.00 | JP-34 | 2.97 | No.1> No. 2 > No. 3 |
| JP-10 | 17.76 | JP-18 | 9.00 | JP-32 | 3.2 | No.1> No. 2 > No. 3 |
| JP-22 | 15.34 | JP-33 | 5.45 | JP-31 | 5.3 | No.1> No. 2 > No. 3 |
| JP-13 | 14.39 | JP-12 | 4.66 | JP-4 | 2.95 | No.1> No. 2 > No. 3 |
| JP-20 | 13.98 | JP-7 | 4.63 | JP-5 | 3.88 | No.1> No. 2 > No. 3 |

**Example 2**

This example provides an evaluation method for the spicy aroma note of cigarette smoke. The specific steps were the same as Example 1, except that the GC-MS analysis conditions were as follows.

Conditions for GC-MS analysis

Chromatographic column: HP-5MS (30 m $\times$ 0.25 mm $\times$ 0.25 $\mu$m) capillary column; inlet temperature: 240°C; transmission line temperature: 280°C; carrier gas: He, with a flow rate of 0.8 mL/min; temperature programming: the temperature was maintained at 40°C for 1.3 min, heated to 155°C at a rate of 6°C/min and maintained for 2.4 min, and then heated to 255°C at a rate of 6°C/min and maintained for 16 min; injection volume: 2 $\mu$L, with a split ratio of 25:1; EI ionization energy: 70 eV; ion source temperature: 230°C; quadrupole temperature: 160°C; mass range: 35-455 amu. The retrieval and qualitative analysis were performed by using the NIST 17 and Wiley 275 standard mass spectral libraries, and the content of partial aroma-contributing components was determined using the internal standard method.

**Example 3**

This example provides an evaluation method for the spicy aroma note of cigarette smoke. The specific steps were the same as Example 1, except that the GC-MS analysis conditions were as follows.

Conditions for GC-MS analysis

Chromatographic column: HP-5MS (30 m $\times$ 0.25 mm $\times$ 0.25 $\mu$m) capillary column; inlet temperature: 240°C; transmission line temperature: 280°C; carrier gas: He, with a flow rate of 1.2 mL/min; temperature programming: the temperature was maintained at 60°C for 0.7 min, heated to 175°C at a rate of 10°C/min and maintained for 1.4 min, and then heated to 275°C at a rate of 10°C/min and maintained for 12 min; injection volume: 2 $\mu$L, with a split ratio of 25:1; EI ionization energy: 70 eV; ion source temperature: 230°C; quadrupole temperature: 160°C; mass range: 35-455 amu. The retrieval and qualitative analysis were performed by using the NIST 17 and Wiley 275 standard mass spectral libraries, and the content of partial aroma-contributing components was determined using the internal standard method.

**Example 4**

This example provides an evaluation method for the spicy aroma note of cigarette smoke. The specific steps were the same as Example 1, except that the chromatographic column HP-5MS was replaced with DB-35MS.

**Example 5**

This example provides an evaluation method for the spicy aroma note of cigarette smoke. The specific steps were the same as Example 1, except that the temperature programming was as follows:
the temperature was maintained at 70°C for 1 min, heated to 210°C at a rate of 24°C/min and maintained for 1 min, and then heated to 280°C at a rate of 6°C/min and maintained for 5 min.

## Example 6

This example provides an evaluation method for the spicy aroma note of cigarette smoke. The specific steps were the same as Example 1, except that neophytadiene was not contained in the aroma-contributing component.

## Example 7

This example provides an evaluation method for the spicy aroma note of cigarette smoke. The specific steps were the same as Example 1, except that methyl linolenate and methyl palmitate were not contained in the aroma-contributing component.

## Example 8

This example provides an evaluation method for the spicy aroma note of cigarette smoke. The specific steps were the same as Example 1, except that cyclopentanone and 3-pentene-2-one were not contained in the aroma-contributing component.

## Example 9

This example provides an evaluation method for the intensity of the spicy aroma note of cigarette smoke. The specific steps were the same as Example 1, except that pyridine was not contained in the aroma-contributing component.

## Example 10

This example provides an evaluation method for the intensity of the spicy aroma note of cigarette smoke. The specific steps were the same as Example 1, except that 3-hydroxybutan-2-one was not contained in the aroma-contributing component.

## Effectiveness Test

An additional seven groups of cigarette samples with varying intensities of the spicy aroma note of cigarette smoke were selected. A sensory panel composed of twelve experts conducted a comparative evaluation in accordance with the provisions of GB 5606.4-2005 "Cigarettes - Technical Requirements for Sense Taste" and determined that the intensity was ranked from lowest to highest as A, B, C, D, E, F, and G. Subsequently, the methods provided in Examples 1-10 were applied to these seven groups of cigarettes for grading, and the Q value was calculated. The results are as follows.

| Group | Q value | | | | | | |
|---|---|---|---|---|---|---|---|
| | A | B | C | D | E | F | G |
| Example 1 | 19.23 | 17.88 | 15.34 | 5.00 | 4.63 | 2.97 | 2.92 |
| Example 2 | 23.47 | 21.84 | 18.73 | 6.10 | 5.64 | 3.62 | 3.61 |
| Example 3 | 13.55 | 11.84 | 10.69 | 4.20 | 4.18 | 2.24 | 2.22 |
| Example 4 | 14.66 | 13.62 | 11.70 | 3.54 | 3.82 | 2.27 | 2.25 |
| Example 5 | 24.31 | 22.55 | 19.37 | 6.31 | 5.81 | 3.69 | 3.71 |
| Example 6 | 9.06 | 8.99 | 9.25 | 2.36 | 2.58 | 1.50 | 1.53 |
| Example 7 | 10.31 | 8.94 | 6.20 | 2.13 | 2.70 | 1.53 | 1.45 |
| Example 8 | 26.01 | 24.83 | 19.97 | 7.12 | 6.59 | 3.94 | 4.20 |
| Example 9 | 36.64 | 30.31 | 31.46 | 8.82 | 7.72 | 5.26 | 4.71 |
| Example 10 | 19.09 | 17.83 | 15.24 | 4.94 | 4.55 | 2.92 | 2.91 |

From the above results, it can be found that in the present application, by controlling specific chromatographic parameters and selecting a specific combination of aroma-contributing components, the spicy aroma note of the smoke of the cigarette to be tested can be more accurately, comprehensively, and effectively evaluated, effectively improving the accuracy of the evaluation results.

The applicant declares that the present application illustrates the evaluation method for the spicy aroma note of cigarette smoke and use thereof of the present application by the above examples, but the present application is not limited to the above examples, that is, the present application does not necessarily rely on the above examples to be implemented. Those skilled in the art should understand that any improvements of the present application, the equivalent substitution of each raw material, the addition of auxiliary ingredients, and the selection of specific methods shall fall within the protection scope and disclosure scope of the present application.

The above describes in detail the preferred embodiments of the present application. However, the present application is not limited to the specific details in the above embodiments, and various simple variations of the technical solutions of the present application can be made within the scope of the technical conception of the present application, all of these simple variations shall fall within the protection scope of the present application.

It is also to be noted that the various specific technical features described in the above specific embodiments may be combined in any suitable manners without contradiction, and in order to avoid unnecessary repetition, the various possible combinations are not described separately in the present application.

## Claims

1. An evaluation method for the spicy aroma note of cigarette smoke, comprising the following steps:

    (1) collecting a mainstream smoke total particulate matter of a typical spicy-type cigarette and a typical non-spicy-type cigarette, performing GC-MS detection to obtain a content of an aroma-contributing component in the typical spicy-type cigarette and the typical non-spicy-type cigarette; and conducting partial least squares-discriminant analysis on the content of the aroma-contributing component to obtain a VIP value of the aroma-contributing component;
    (2) collecting a mainstream smoke total particulate matter of a cigarette to be tested, and performing GC-MS detection to obtain a content of an aroma-contributing component in the cigarette to be tested; and
    (3) calculating a spicy aroma note index Q for cigarette smoke of the cigarette to be tested based on the VIP value of the aroma-contributing component from step (1) and the content of the aroma-contributing component in the cigarette to be tested obtained in step (2), and determining a degree of the spicy aroma note of cigarette smoke of the cigarette to be tested;
    steps (1) and (2) are not required to be performed in a particular order;
    the aroma-contributing component comprises a positively correlated aroma-contributing component and a negatively correlated aroma-contributing component;
    the positively correlated aroma-contributing component comprises any one or a combination of at least two of a terpenoid aroma-contributing component, a long-chain unsaturated fatty acid methyl ester, and a positively correlated ketonic aroma-contributing component; and the negatively correlated aroma-contributing component comprises any one or a combination of at least two of a nitrogen-containing heterocyclic aroma-contributing component, a furanic aroma-contributing component, and a negatively correlated ketonic aroma-contributing component.

2. The evaluation method according to claim 1, wherein the positively correlated aroma-contributing component comprises a combination of a terpenoid aroma-contributing component, a long-chain unsaturated fatty acid methyl ester, and a positively correlated ketonic aroma-contributing component;
    optionally, the negatively correlated aroma-contributing component comprises a combination of a nitrogen-containing heterocyclic aroma-contributing component, a furanic aroma-contributing component, and a negatively correlated ketonic aroma-contributing component.

3. The evaluation method according to claim 1 or 2, wherein the terpenoid aroma-contributing component comprises neophytadiene;

    optionally, the long-chain unsaturated fatty acid methyl ester aroma-contributing component comprises methyl linolenate and/or methyl palmitate;
    optionally, the positively correlated ketonic aroma-contributing component comprises 3-hydroxybutan-2-one.

4. The evaluation method according to any one of claims 1-3, wherein the nitrogen-containing heterocyclic aroma-contributing component comprises pyridine;

optionally, the furanic aroma-contributing component comprises 2-ethylfuran;
optionally, the negatively correlated ketonic aroma-contributing component comprises cyclopentanone and/or 3-pentene-2-one.

5.  The evaluation method according to claim 3 or 4, wherein the aroma-contributing component comprises neophytadiene, methyl linolenate, methyl palmitate, 3-hydroxybutan-2-one, pyridine, 2-ethylfuran, cyclopentanone, and 3-pentene-2-one.

6.  The evaluation method according to any one of claims 1-5, wherein in the GC-MS detection, a chromatographic column is HP-5MS.

7.  The evaluation method according to any one of claims 1-6, wherein in the GC-MS detection, a carrier gas flow rate is 0.8-1.2 mL/min.

8.  The evaluation method according to any one of claims 1-7, wherein in the GC-MS detection, a temperature program is as follows:
    setting 40-60°C as an initial temperature and maintaining for 0.7-1.3 min; heating to 155-175°C at a rate of 6-10°C/min and maintaining for 1.4-2.4 min; and then heating to 255-275°C at a rate of 6-10°C/min and maintaining for 12-16 min.

9.  The evaluation method according to any one of claims 1-8, wherein a calculation formula of the spicy aroma note index Q in step (3) is as follows:

    $Q = \Sigma$(content of positively correlated aroma-contributing component i $\times$ VIP$_i$) / $\Sigma$(content of negatively correlated aroma-contributing component j $\times$ VIP$_j$);
    wherein VIP$_i$ refers to the VIP value of the positively correlated aroma-contributing component i, and VIP$_j$ refers to the VIP value of the negatively correlated aroma-contributing component j.

10. Use of the evaluation method according to any one of claims 1-9 in the evaluation and analysis of aroma note of cigarette smoke.

TRANSLATION

## INTERNATIONAL SEARCH REPORT

International application No.

**PCT/CN2025/103355**

| A. | CLASSIFICATION OF SUBJECT MATTER |
|---|---|

G01N30/02(2006.01)i; G01N30/72(2006.01)i; G01N30/86(2006.01)i; G06F17/11(2006.01)i

According to International Patent Classification (IPC) or to both national classification and IPC

| B. | FIELDS SEARCHED |
|---|---|

Minimum documentation searched (classification system followed by classification symbols)

IPC:G01N G06F

Documentation searched other than minimum documentation to the extent that such documents are included in the fields searched

Electronic data base consulted during the international search (name of data base and, where practicable, search terms used)

CNTXT, ENTXTC, WPABSC, VEN, ENTXT, CJFD, CNKI, ISI Web of Science: 云南中烟, 卷烟, 烟, 辛香, 香型, 香韵, 新植二烯, 正相关, 负相关, GC, MS, pungent, spicy, cigarette, smoke, aroma, negative, positive

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 119355154 A (CHINA TOBACCO YUNNAN INDUSTRIAL CO., LTD.) 24 January 2025 (2025-01-24)<br>    claims 1-10 | 1-10 |
| Y | CN 105241976 A (CHINA TOBACCO YUNNAN INDUSTRIAL CO., LTD.) 13 January 2016 (2016-01-13)<br>    description, paragraphs [0004]-[0096] | 1-10 |
| Y | CN 106093246 A (CHINA TOBACCO YUNNAN INDUSTRIAL CO., LTD.) 09 November 2016 (2016-11-09)<br>    description, paragraphs [0004]-[0041] | 1-10 |
| Y | CN 117044983 A (CHINA TOBACCO YUNNAN INDUSTRIAL CO., LTD.) 14 November 2023 (2023-11-14)<br>    description, paragraphs [0072]-[0079] | 3-5 |
| A | CN 115792022 A (CHINA TOBACCO JIANGSU INDUSTRIAL CO., LTD.) 14 March 2023 (2023-03-14)<br>    entire document | 1-10 |

☑ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 August 2025** | **21 August 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

### INTERNATIONAL SEARCH REPORT

| International application No. |
|---|
| **PCT/CN2025/103355** |

| C. | DOCUMENTS CONSIDERED TO BE RELEVANT | | |
|---|---|---|
| Category* | Citation of document, with indication, where appropriate, of the relevant passages | Relevant to claim No. |
| A | CN 111289677 A (ZHENGZHOU TOBACCO RESEARCH INSTITUTE OF CNTC) 16 June 2020 (2020-06-16)<br>entire document | 1-10 |
| A | CN 113376268 A (CHINA TOBACCO HEBEI INDUSTRY CO., LTD.) 10 September 2021 (2021-09-10)<br>entire document | 1-10 |

Form PCT/ISA/210 (second sheet) (July 2022)

TRANSLATION

**INTERNATIONAL SEARCH REPORT**
**Information on patent family members**

International application No.

**PCT/CN2025/103355**

| Patent document cited in search report | | | Publication date (day/month/year) | Patent family member(s) | Publication date (day/month/year) |
|---|---|---|---|---|---|
| CN | 119355154 | A | 24 January 2025 | None | |
| CN | 105241976 | A | 13 January 2016 | None | |
| CN | 106093246 | A | 09 November 2016 | None | |
| CN | 117044983 | A | 14 November 2023 | None | |
| CN | 115792022 | A | 14 March 2023 | None | |
| CN | 111289677 | A | 16 June 2020 | None | |
| CN | 113376268 | A | 10 September 2021 | None | |

Form PCT/ISA/210 (patent family annex) (July 2022)

**REFERENCES CITED IN THE DESCRIPTION**

**Non-patent literature cited in the description**

- Cigarette - Determination of total and nicotine-free dry particulate matter using a routine analytical smoking machine. *GB/T 19609-2004*

- Cigarettes - Technical Requirements for Sense Taste. *GB 5606.4-2005*